Europäisches Patentamt

European Patent Office

Office européen des brevets

(11) Publication number: **0 327 945**
**A2**

# EUROPEAN PATENT APPLICATION

(21) Application number: **89101720.4**

(22) Date of filing: **01.02.89**

(51) Int. Cl.⁴: **C12P 13/08** , //(C12P13/08, C12R1:15)

The applicant has filed a statement in accordance with Rule 28 (4) EPC (issue of a sample only to an expert). Accession number(s) of the deposit(s): FERM BP-158, FERM BP-1069, FERM BP-1655.

(30) Priority: **03.02.88 JP 23542/88**

(43) Date of publication of application:
**16.08.89 Bulletin 89/33**

(84) Designated Contracting States:
**DE ES FR GB**

(71) Applicant: **KYOWA HAKKO KOGYO CO., LTD.**
**6-1, Ohte-Machi Itchome**
**Chiyoda-ku Tokyo(JP)**

(72) Inventor: **Hirao, Toshihiko**
**2-2-201, Kyowa-cho**
**Hofu-shi Yamaguchi-ken(JP)**
Inventor: **Nakano, Tetsuo**
**2-10-104, Kyowa-cho**
**Hofu-shi Yamaguchi-ken(JP)**
Inventor: **Azuma, Tomoki**
**2-2-303, Kyowa-cho**
**Hofu-shi Yamaguchi-ken(JP)**
Inventor: **Nakanishi, Toshihide**
**2-2-405, Kyowa-cho**
**Hofu-shi Yamaguchi-ken(JP)**

(74) Representative: **Vossius & Partner**
**Siebertstrasse 4 P.O. Box 86 07 67**
**D-8000 München 86(DE)**

(54) Process for producing L-lysine.

(57) Disclosed is a process for producing L-lysine, which comprises culturing in a medium a coryneform glutamic acid-producing microorganism which exhibits resistance to an iturin-related substance and the ability to produce L-lysine, accumulating L-lysine in the culture, and recovering L-lysine therefrom.

EP 0 327 945 A2

# PROCESS FOR PRODUCING L-LYSINE

The present invention relates to a process for producing L-lysine by fermentation.

L-lysine has various uses in the fields of animal feed, medicament, food, etc.

Heretofore, processes for producing L-lysine using various nutrient-requiring strains, various drug-resistant strains, various drug-sensitive strains of coryneform glutamic acid-producing microorganisms have been known. Furthermore, a process using antibiotic-resistant strains (US Patent No. 3,687,810), and a process using strains having resistance to antibacterial substances produced by microorganisms of the genus Bacillus [Japanese Published Unexamined Patent Application No. 169497/84] are known.

However, no report has been made of strains having resistance to iturin-related substances. The object of the process using strains having a resistance to the antibacterial substances produced by microorganisms of the genus Bacillus, disclosed in Japanese Published Unexamined Patent Application No. 169497/84, is not to increase the lysine productivity of the lysine-producing microorganisms, but to prevent growth inhibition of the lysine-producing microorganisms because of contamination of microorganisms of the genus Bacillus.

It has been desired to develop a process for producing, at an industrially lower cost, L-lysine.

The present inventors have made extensive studies on a process for producing L-lysine by fermentation in order to obtain L-lysine at an industrially low cost, and consequently have found that the productivity of L-lysine-producing microorganisms can be increased by conferring resistance to iturin-related substances on coryneform glutamic acid-producing microorganism.

The present invention provides a process for producing L-lysine, which comprises culturing in a medium a coryneform glutamic acid-producing microorganism exhibiting a resistance to an iturin-related substance and the ability to produce L-lysine, accumulating L-lysine in the culture, and recovering L-lysine therefrom.

The coryneform glutamic acid-producing microorganism herein referred to is a microorganism belonging to the genus Corynebacterium, Brevibacterium, Microbacterium or Arthrobacter.

The iturin-related substance herein referred to is an antibiotic produced by microorganisms of the genus Bacillus and includes, for example, iturin A, iturin C, mycosubtilin, bacillomycin L [Biochim. Biophys. Acta 552, 558-562 (1979), Biochim. Biophys. Acta 684, 207-211 (1982)], and a mixture thereof.

As parent strains of the present mutant strains, any of strains belonging to coryneform glutamic acid-producing microorganisms, having an ability to produce lysine, may be used. Specifically Corynebacterium glutamicum H-3149 (FERM BP-158), and Corynebacterium glutamicum H-4412 (FERM BP-1069) are mentioned.

Mutant strains having resistance to an iturin-related substance can be obtained by treating the aforementioned parent strain with 250 μg/mℓ N-methyl-N′-nitro-N-nitrosoguanidine at 30° C for 30 minutes, isolating the strains growing on a minimum medium agar plate containing the iturin-related substance at such a concentration that the parent strains can not grow. Further, the mutant strains of the present invention can be obtained by culturing the thus obtained mutant strains having resistance to the iturin-related substance, making a comparison of the ability to produce L-lysine between the strains and selecting the strains with an increased ability to produce L-lysine.

The above-mentioned parent strains and mutant strains having resistance to an iturin-related substance (a 1:1 mixture of iturin A and iturin C) derived therefrom have the following properties:

Parent strain:

Corynebacterium glutamicum H-3149 (FERM BP-158) having S-(2-aminoethyl)cysteine-resistance, rifampicin-resistance, streptomycin-resistance and 6-azauracil-resistance. Mutant strain:

Corynebacterium glutamicum H-4933 having S-(2-aminoethyl)cysteine-resistance, rifampicin-resistance, streptomycin-resistance, 6-azauracil-resistance, and iturin-related substance-resistance. Parent strain:

Corynebacterium glutamicum H-4412 (FERM BP-1069) having S-(2-aminoethyl)cysteine-resistance, rifampicin-risistance, streptomycin-resistance, 6-azauracil-resistance and β-naphthoquinoline-resistance.

Mutant strain:

Corynebacterium glutamicum H-4934 having S-(2-aminoethyl)cysteine-resistance, rifampicin-resistance, streptomycin-resistance, 6-azauracil-resitance, β-naphthoquinoline-resistance and iturin-related substance-resistance.

The thus obtained iturin-related substance-resistant mutant strain Corynebacterium glutamicum H-4934 was deposited on January 14, 1988 with the Fermentation Research Institute, Agency of Industrial Science and Technology Japan (FRI), under deposition number of FERM BP-1655.

Growth degree of the afore-mentioned strains is shown in Table 1 when cultured on a minimum agar plate [glucose: 10 g/$\ell$, ammonium chloride: 1 g/$\ell$, urea: 2 g/$\ell$, KH$_2$PO$_4$: 1 g/$\ell$, K$_2$HPO$_4$: 3 g/$\ell$, MgCl$_2$·2H$_2$O: 0.4 g/$\ell$, FeSO$_4$·7H$_2$O: 10 mg/$\ell$, MnSO$_4$·4 H$_2$O: 10 mg/$\ell$, ZnSO$_4$·7H$_2$O: 1 mg/$\ell$, CuSO$_4$·5H$_2$O: 1 mg/$\ell$, (NH$_4$)$_6$Mo$_7$O$_{24}$·4H$_2$O: 1 mg/$\ell$, d-biotin: 50 $\mu$g/$\ell$, agar: 20 g/$\ell$, pH 7.2] containing 200 $\mu$g/m$\ell$ iturin (a 1:1 mixture of iturin A and iturin C) for 24 hours.

Table 1

| Iturin ($\mu$g/m$\ell$) | H-3149 | H-4412 | H-4933 | H-4934 |
|---|---|---|---|---|
| 0 | + + | - | + + | + + |
| 200 | - | - | + + | + + |
| + +: sufficient growth -: no growth | | | | |

For the culturing of the microorganisms suitable in the present invention, media as used in the fermentation production of amino acids may be usually used. That is, any synthetic medium or a natural medium may be used so long as it properly contains carbon sources, nitrogen sources, inorganic salts, growth factors, etc. which can be assimilated by the microorganisms.

As the carbon sources, saccharide such as glucose, fructose, sucrose, molasses, starch hydrolyzate, etc., organic acids such as acetic acid, fumaric acid, citric acid, etc. and alcohols such as ethanol, glycerol, mannose, etc. may be used.

As the nitrogen sources, ammonia, ammonium salts of various inorganic or organic acids such as ammonium chloride, ammonium sulfate, ammonium acetate, ammonium phosphate, etc., amines, other nitrogen-containing compounds, peptone, meat extract, corn steep liquor, casein hydrolyzate, soybean cake hydrolyzate, various fermentation cell and their digested products may be used.

As the inorganic compounds, potassium dihydrogen phosphate, dipotassium hydrogen phosphate, magnesium phosphate, magnesium sulfate, sodium chloride, ferrous sulfate, manganese sulfate, copper sulfate, calcium carbonate, etc. may be used.

In addition, vitamins such as biotin, thiamine, nicotinic acid, $\beta$-alanine, pantothenic acid, etc., amino acids such as leucine, valine, aspartic acid, glutamic acid, etc., and various antibiotics such as streptomycin, etc. may be added thereto, when requried.

Culturing is carried out under aerobic conditions, for example, by shaking culturing or aeration-agitation submarged culture etc. at a culturing temperature of 20 to 40°C, preferably 25 to 38°C. The pH of the medium is in the range of 5 to 9, and is preferably kept at around neutrality. The pH adjustment of the medium is carried out with calcium carbonate, inorganic or organic acids, alkali solutions, ammonia or pH buffer solutions.

Usually after culturing continues for 1 to 7 days, L-lysine is produced and accumulated in the culture.

After the completion of culturing, precipitates such as cells, etc. are removed from the culture and L-lysine can be recovered therefrom by simultaneous application of ion exchange treatment, concentration, salting-out, isoelectric point precipitation, etc.

Example of the present invention will be given below.

Example 1

Corynebacterium glutamicum H-3149, H-4412, H-4933 or H-4934 was inoculated into a 300 m$\ell$ Erlenmeyer flask containing 20 m$\ell$ of the following seed medium and subjected to shaking culture (220 rpm) at 30°C for 24 hours. Then, 2 m$\ell$ of the seed culture was inoculated into a 300 m$\ell$ Erlenmeyer flask containing 20 m$\ell$ of the following production medium and subjected to shaking culture (220 rpm) at 30°C for 72 hours. After the completion of culturing, the amount of produced L-lysine was measured by colorimetry using acidic copper ninhydrine. It was found that the amount of produced L-lysine in the culture was 44 mg/m$\ell$, 52 mg/m$\ell$, 53 mg/m$\ell$ and 54 mg/m$\ell$, respectively in terms of hydrochloride. Then, 1000 m$\ell$ of the culture obtained with the strain H-4933 was centrifuged and the supernatant thus obtained was adjusted to pH 1.5 with sulfuric acid and then passed through a column of highly acidic ion exchange resin Diaion SK-1B (H$^+$-type, product of Mitsubishi Kasei Corporation) to adsorb L-lysine. Then, the column was washed with water and subjected to elution with 2N aqueous ammonia to collect the L-lysine fractions. The collected fractions were concentrated and the concentrated solution was adjusted to pH 2.0 with hydrochloric acid and cooled while adding ethanol thereto, whereby L-lysine hydrochloride was crystallized. The amount of the crystal was 42 g.

| Seed medium (pH 7.2) | | Production medium (pH 7.2) | |
|---|---|---|---|
| Glucose | 40 g/$\ell$ | Blackstrap molasses (in terms of glucose) | 100 g/$\ell$ |
| Peptone | 20 g/$\ell$ | | |
| Yeast extract | 5 g/$\ell$ | $KH_2PO_4$ | 0.5 g/$\ell$ |
| Urea | 3 g/$\ell$ | Ammonium sulfate | 40 g/$\ell$ |
| $KH_2PO_4$ | 1.5 g/$\ell$ | $MgSO_4 \cdot 2H_2O$ | 0.5 g/$\ell$ |
| $K_2HPO_4$ | 0.5 g/$\ell$ | Urea | 3 g/$\ell$ |
| $MgSO_4 \cdot 2H_2O$ | 0.5 g/$\ell$ | Calcium carbonate | 30 g/$\ell$ |
| Biotin | 50 $\mu$g/$\ell$ | | |

## Claims

1. A process for producing L-lysine, which comprises culturing in a medium a coryneform glutamic acid-producing microorganism, which exhibits resistance to an iturin-related substance and the ability to produce L-lysine, accumulating L-lysine in the culture, and recovering L-lysine therefrom.

2. The process according to claim 1, wherein the iturin-related substance is iturin A, iturin C, mycosubtilin, bacillomycin L or a mixture thereof.

3. The process according to claim 1 or 2, wherein the micro-organism is Corynebacterium glutamicum H-4933 or Corynebacterium glutamicum H-4934 (FERM BP-1655).

4. A biologically pure culture of Corynebacterium glutamicum H-4933.

5. A biologically pure culture of Corynebacterium glutamicum H-4934 (FERM BP-1655).

4